# EUROPEAN PATENT APPLICATION

(11) **EP 3 905 186 A1**
(43) Date of publication of application: **03.11.2021**
(21) Application number: 18944700.6
(22) Date of filing: 26.12.2018
(51) Int. Cl.: G06Q 50/02, G06Q 50/10

(54) **INSECT BREEDING CONTROL DEVICE AND METHOD**

(71) Applicant: Foodyworm. Inc., Chungcheongbuk-do 27867 (KR)
(72) Inventor: KIM, Tae Hoon, Cheongju-si Chungcheongbuk-do 28160 (KR); LEE, You Seok, Cheongju-si Chungcheongbuk-do 28160 (KR); JOO, Yeong Pyo, Cheongju-si Chungcheongbuk-do 28160 (KR); SHIN, Dae Cheol, Cheongju-si Chungcheongbuk-do 28160 (KR); LEE, Hye Jin, Cheongju-si Chungcheongbuk-do 28160 (KR); KWON, Min Jeong, Cheongju-si Chungcheongbuk-do 28160 (KR)
(74) Representative: BRP Renaud & Partner mbB Rechtsanwälte Patentanwälte Steuerberater
(86) International application number: PCT/KR2018/016667
(87) International publication number: WO 2020/138526

(57) **Abstract**

The present invention relates to a technology for breeding an insect. A breeding control device comprises: a database which stores a combination of environmental factors that are set in consideration of the growth of a feed insect and the nutrient after feed processing; a plurality of sensors which detect an environmental factor relating to a farm for breeding a feed insect and a growth and development state of the insect; a processing unit which receives an input on expected demand and shipment time of feed produced from the feed insect, calculates a growth rate of the feed insect, required from the current farm according to the input expected demand and shipment time and the detected growth and development state of the insect, and reads a combination of environmental factors from the database in consideration of the environmental factor sensed through the sensors and the calculated growth rate; and a control unit which controls the growth of the feed insect in the farm by adjusting a growth and development control factor corresponding to each environmental factor according to the combination of environmental factors read out from the processing unit.

## Description

### [Technical Field]

The present disclosure relates to a technology for breeding insects by artificially controlling various environmental conditions in a breeding farm, and more particularly, a device and method for controlling insect breeding by monitoring the growth of insects and controlling environmental conditions to promote breeding in an Internet of Things (IoT)-based breeding farm combined with sensors and control means, and a recording medium for recording the method.

### [Background Art]

In 2012, at a Food Security Advisory Meeting held in Rome, Italy by the headquarters of Food and Agriculture Organization (FAO) of the United Nations, the potential advantages of insect food and feed use were announced as one of the strategies for achieving global food security. It was emphasized that insects are very efficient compared to feed in food production. As illustrated, there is a difference in efficiency between insects and livestock. For insects, 1 kg of insect food can be made with 2 kg of feed. However, for livestock, 1 kg of meat can be obtained only when 8 kg of feed is eaten.

According to data from the Ministry of Agriculture, Food and Rural Affairs and the Rural Development Administration, the global market for the insect industry is expected to reach 38 trillion won in 2020, which corresponds to a 345% increase from 2007. In particular, the compound annual growth rate (CAGR) of the domestic market is 26.5%, indicating that the insect industry is a very high-growth industry compared to other businesses. As such, the demand for stable feed insect products with high quality and nutritional value is increasing in the companion animal market or the pet market, and the sales scale is also increasing rapidly.

However, there is a lack of standardized breeding manuals for feed insects such as *Ptecticus tenebrifer* in breeding farms, and technology to cope with accidents such as death of all insets when exposed to natural enemies such as mold without protection is insufficient. There are also difficulties in securing sufficient supply.

Internet of Things technology is a technology that can bring about other fundamental changes in our lives by enabling communication between people and things, rather than simply connecting things over a network. Traditional insect breeding techniques have accepted the delayed response to the situation as fate due to the intermittent situation detection through the senses of each insect breeder, the situation judgment depending on personal knowledge, and time/space constraints. However, with the help of IoT technology, it is expected that many of the conventional technical limitations can be resolved.

### (Prior Art Literature)

### (Patent Document)

Korean Patent No. 0874806, registered on December 11, 2008, titled "INSECT RAISING DEVICE"

### [Disclosure]

### [Technical Problem]

Therefore, the present disclosure has been made in view of the above problems, and it is one object of the present disclosure to overcome a limitation in that the smart farm technology presented through combination of traditional agricultural and the Internet of Things is still at the level of monitoring or simple growth control of plant factories in implementation, and to improve the current situation where the management of insect products for feed raised on the breeding farm relies on the experience and intuition of the farmer.

### [Technical Solution]

In accordance with one aspect of the present disclosure, provided is a breeding control device including a database configured to store a combination of environmental factors set in consideration of growth of feed insects and nutrients after feed processing; a plurality of sensors configured to detect environmental factors and a growth status of the insects related to a breeding farm for breeding the feed insects; a processor configured to receive an input of an expected demand amount and shipment time of feed produced from the feed insects, calculate a growth rate of the feed insects requested from the breeding farm according to the input expected demand amount, the shipment time, and the detected growth status of the insects, and read the combination of the environmental factors from the database in consideration of the environmental factors detected through the sensors and the calculated growth rate; and a controller configured to control the growth of the feed insects on the breeding farm by adjusting a growth control factor corresponding to each of the environmental factors according to the combination of the environmental factors read from the processor.

According to an embodiment, the sensors may include: an environmental sensor configured to detect at least one environmental factor among temperature, illuminance, humidity, and air quality involved in the growth of the insects on the breeding farm; and a growth sensor configured to detect a growth status of at least one of a kind, size, color, movement, and number of the insects.

According to an embodiment, the processor may identify exposure or proliferation of a natural enemy included in an image sensed by the sensors, and transmit a control command for suppressing the natural enemy to the controller. Here, the database may pre-store a critical range of the environmental factors for suppressing growth of a corresponding natural enemy according to each kind of natural enemy corresponding to the feed insects, wherein the processor may query the database for the identified natural enemy, read a combination of environmental factors for suppressing the growth of the natural enemy, and generate the control command in consideration of the growth status of the feed insects. Furthermore, when the natural enemy is identified, the processor may notify the manager of a situation related to the exposure or proliferation of the natural enemy in real time.

According to an embodiment, the processor may specify a movement area from the image sensed through the sensors, wherein the processor may be configured to identify insects or natural enemies corresponding to the movement area by comparing the specified movement area with a preset size, color, or movement pattern of the feed insects or the natural enemy corresponding to the feed insects, or a combination thereof, and extract detailed information about the growth status by determining a kind of the identified insect or natural enemy, counting the number of the identified insects or natural enemies for each determined kind of insects or natural enemies, measuring a size of the insects or natural enemies, or quantifying a degree of movement of the insects or natural enemies. In addition, when the specified movement area matches only a part of preset characteristic information about the feed insects, the processor may check whether the area matches additional information indicating a disease state of the feed insects and determine whether the feed insects have a disease. Further, when the counted number of the insects for each kind of the insects is greater than or equal to a space threshold in an allocated space on the breeding farm, the processor may calculate an additional space required for growth of the insects and notify a manager of the calculated space.

According to an embodiment, the processor may be configured to calculate a difference between the input expected demand and an amount of insects to be supplied from the breeding farm, calculate remaining days to the input shipment time, and calculate a growth rate of the feed insects required from the breeding farm based on the difference and the remaining days.

According to an embodiment, in consideration of the calculated growth rate, the processor may fix factors not involved in the growth of the feed insects among the environmental factors detected through the sensors, search the database while changing the factors capable of changing the growth of the feed insects, and read the combination of the environmental factors for the breeding farm.

According to an embodiment, the database may store a combination of environmental factors experimentally set for growth of the feed insects such that target remaining nutrients are maximized after the feed processing through roasting and drying.

In accordance with another aspect of the present disclosure, provided is a breeding control method including: storing, in a database, a combination of environmental factors set in consideration of a growth of feed insects and nutrients after feed processing; detecting environmental factors and a growth status of the insects related to a breeding farm for breeding the feed insects using a plurality of sensors; receiving an input of an expected demand amount and shipment time of feed produced from the feed insects, calculating a growth rate of the feed insects requested from the breeding farm according to the input expected demand amount, the shipment time, and the detected growth status of the insects, and reading the combination of the environmental factors from the database in consideration of the environmental factors detected through the sensors and the calculated growth rate; and controlling the growth of the feed insects on the breeding farm by adjusting a growth control factor corresponding to each of the environmental factors according to the read combination of the environmental factors.

According to an embodiment, the method may further include: identifying exposure or proliferation of a natural enemy included in an image sensed by the sensors; and transmitting a control command for suppressing the natural enemy to the breeding farm. According to an embodiment, the method may further include: pre-storing, in the database, a critical range of the environmental factors for suppressing a growth of a corresponding natural enemy according to each kind of natural enemy corresponding to the feed insects, wherein the transmitting of the control command to the breeding farm may include: querying the database for the identified natural enemy; reading a combination of environmental factors for suppressing the growth of the natural enemy; and generating the control command in consideration of the growth status of the feed insects.

According to an embodiment, the reading of the combination of the environmental factors may include: specifying a movement area from the image sensed through the sensors; identifying insects or natural enemies corresponding to the movement area by comparing the specified movement area with a preset size, color, or movement pattern of the feed insects or the natural enemy corresponding to the feed insects, or a combination thereof; and extracting detailed information about the growth status by determining a kind of the identified insect or natural enemy; counting the number of the identified insects or natural enemies for each determined kind of insects or natural enemies; measuring a size of the insects or natural enemies; or quantifying a degree of movement of the insects or natural enemies. When the specified movement area matches only a part of preset characteristic information about the feed insects, the identifying of the insects or natural enemies may include checking whether the area matches additional information indicating a diseased state of the feed insects and determining whether the feed insects have a disease. The method may further include, when the counted number of the insects for each kind of the insects is greater than or equal to a space threshold in an allocated space on the breeding farm, calculating an additional space required for growth of the insects and notifying a manager of the calculated space.

According to an embodiment, the reading of the combination of the environmental factors may include: calculating a difference between the input expected demand and an amount of insects to be supplied from the breeding farm; calculating remaining days to the input shipment time; and calculating a growth rate of the feed insects required from the breeding farm based on the difference and the remaining days.

According to an embodiment, in consideration of the calculated growth rate, the reading of the combination of the environmental factors may include: fixing factors not involved in the growth of the feed insects among the environmental factors detected through the sensors; searching the database while changing the factors capable of changing the growth of the feed insects; and reading the combination of the environmental factors for the breeding farm.

Provided herein is a computer-readable recording medium having a program for executing the above-described breeding control method in a computer recorded thereon.

### [Advantageous Effects]

According to embodiments of the present disclosure, the competitiveness of the insect industry may be enhanced by controlling the growth rate of insects in consideration of the supply and demand of feed insects, as well as environmental factors for promotion of insect breeding. Also, high-quality feed insects may be supplied by controlling the environmental factors necessary for growth from the perspective of food and nutrition after feed processing. In addition, by monitoring the activity of insects or natural enemies through various sensors and image identification technology provided in the breeding farm and performing automated real-time control, early prediction of insect growth status and a proper action may be enabled.

### [Description of Drawings]

FIG. 1 is a view showing a breeding farm and an insect for feed which is a breeding target for implementation of embodiments of the present disclosure.
FIG. 2 is a block diagram illustrating a breeding control device for insects according to an embodiment of the present disclosure.
FIG. 3 is a view showing an overall outline of a breeding system including the breeding control device for insects of FIG. 2 according to an embodiment of the present disclosure.
FIG. 4 exemplarily shows sensors and communication means that can be used in a breeding control device for insects according to an embodiment of the present disclosure.
FIG. 5 illustrates the process of identifying and classifying insects adopted according to embodiments of the present disclosure.
FIGS. 6A and 6B depict the results of monitoring environmental factors when damage is caused by natural enemies.
FIG. 7 is a flowchart illustrating a method for controlling the breeding of insects according to an embodiment of the present disclosure.

### [Best Mode]

Hereinafter, embodiments of the present disclosure will be described in detail with reference to the drawings. In the following description and accompanying drawings, detailed descriptions of well-known functions or elements may be omitted in order not to obscure the subject matter of the present disclosure. Throughout the specification, "including" a certain component does not exclude other components, but means that other components may be further included, unless otherwise stated.

Terms used in this specification are merely adopted to describe specific embodiments, and are not intended to limit the present disclosure. A singular expression may include a plural expression unless the context clearly indicates otherwise. In this specification, "include" or "have" is intended to indicate that characteristics, figures, operations, operations, constituents, and components disclosed in the specification or combinations thereof exist. "Include" or "have" should be understood as not precluding existence or addition of one or more other characteristics, figures, operations, operations, constituents, components, or combinations thereof.

Unless stated otherwise, all terms used herein, including technical or scientific terms, have the same meaning as commonly understood by those of ordinary skill in the art to which the present invention belongs. Terms such as those defined in commonly used dictionaries are to be interpreted as having a meaning consistent with the meaning in the context of the related art and are not to be construed as ideal or overly formal in meaning unless expressly defined in the present application.

As introduced above, stable supply according to the supply and demand of insects for feed is the most important issue in determining the size of the insect market. In particular, in the case of the domestic market, the issue of supply and demand of insect feed has been raised as the most difficult issue for several years. The key to addressing the issue is that, first, insect feed food companies that can sufficiently accept the supply from breeding farms should grow, and second, consistent breeding of insect feed should be performed based on a standardized breeding manual.

IoT-based low-cost container-type breeding facilities are expected to contribute to stable sales of breeding farms as they allow mutual communication and allow real-time transfer of a breeding technique. It is also expected that consumer satisfaction will be greatly increased in the process of commercialization and distribution of the insect feed as it is possible to operate the real-name system for producers of breeding farms with safe insect breeding technology. In this IoT-based low-cost container-type breeding facility, various internal environmental/control elements are connected by sensors and IoT technology, and thus all acquired information can be provided over a network. In addition, since the control of the breeding environment (feeding cycle, environment conditioning regarding temperature, humidity, and the like, etc.) is automated, breeding costs such as labor costs may be greatly reduced. Also, by providing an optimized breeding environment, the breeding period may be shortened while maximizing the growth and development of insects.

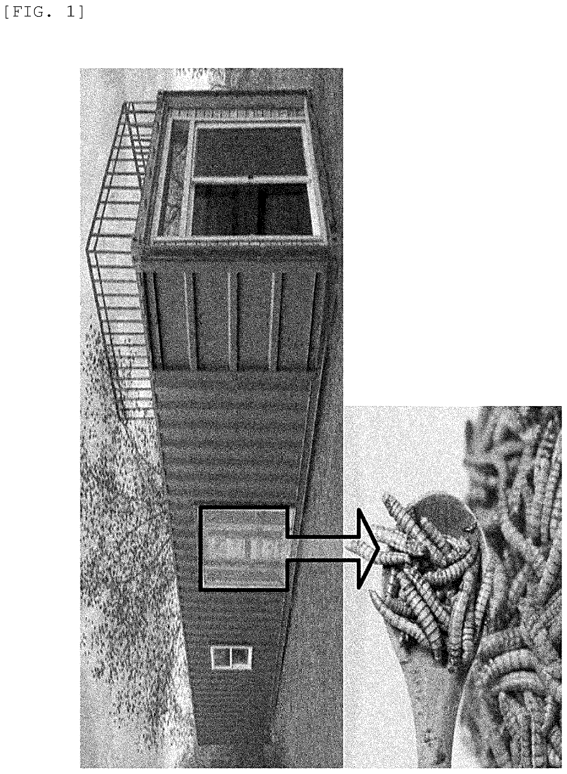

FIG. 1 is a view showing a breeding farm and an insect for feed which is a breeding target for implementation of embodiments of the present disclosure. Feed insects are grown in the breeding farm shown in the figure.

IoT-based smart insect breeding technology may enable continuous and scientific situation detection through various sensors, and enable professional and automated situation determination based on various pre-built knowledge databases on insect breeding. It may also enable a timely response to situations that transcends temporal/spatial constraints. In particular, the integration of IoT-based condition monitoring and condition diagnosis technology is recognized as a groundbreaking attempt to strengthen the competitiveness of the insect industry and change the paradigm of the insect breeding industry as it enables not only an action according to early prediction of diseases and insect growth conditions, but also the production of high-quality and high-value-added insects through insect condition management.

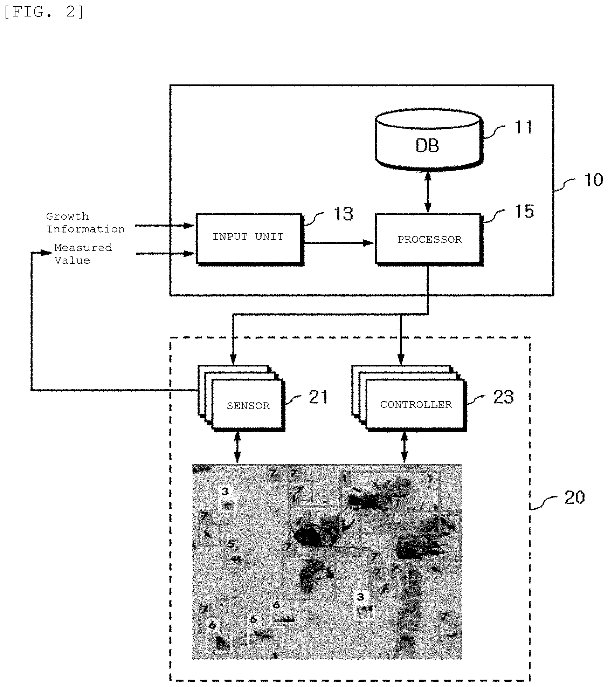

FIG. 2 is a block diagram illustrating a breeding control device for insects according to an embodiment of the present disclosure. The breeding control device may include a S/W module 10 and a H/W module 20. However, the present disclosure is not limited thereto, and may be implemented in various ways according to implementation needs.

The S/W module 10 of the breeding control device may include a database 11 to store various information about insect growth, an input unit 13 to receive growth information or measured values, and a processor 15 to generate a command for monitoring and controlling of the state of the breeding farm. The H/W module 20 of the breeding control device may include a plurality of sensors 21 to detect various environmental factors and growth conditions of the breeding farm and to acquire measured values thereof and a controller 23 to adjust the environmental factors for the breeding farm according to the control command of the processor 15. Each component will be described in more detail below.

The database 11 stores a combination of environmental factors which are set in consideration of the growth of feed insects and nutrients after feed processing. In particular, in embodiments of the present disclosure, the database 11 may store a combination of environmental factors experimentally set for growing the feed insects such that the target remaining nutrients are maximized after feed processing through roasting and drying. Considering that the target of insect growth is not simply to promote growth rate or increase size, but rather to obtain "feed insects," the growth should be set to improve the quality of feed, which is the final product, in feed processing. A combination of environmental factors for growth may be derived, focusing on nutrient components to be enhanced in feed insects, which are raw materials processed into feed through a series of processes such as washing, drying, roasting, and pulverization, and may be stored in the database 11 to be used later for control of the breeding farm.

The plurality of sensors 21 is configured to detect the environmental factors related to the breeding farm for breeding the feed insects, and the growth status of the insects. The sensors 21 may include an environmental sensor to detect at least one of temperature, illuminance, humidity, and air quality, which are environmental factors involved in the growth of insects on the breeding farm, and a growth sensor to detect at least one of the kind, size, color, movement and population of the insects, which are growth statuses.

The processor 15 receives an input of the expected demand amount and shipment time of the feed produced from the feed insects, and calculates the growth rate of the feed insects requested from the current breeding farm according to the input expected demand amount, the shipment time, and the detected growth status of the insects. It also reads the combination of the environmental factors from the database in consideration of the environmental factors detected through the sensors and the calculated growth rate, and generates a control command therefrom and delivers the command to the controller 23.

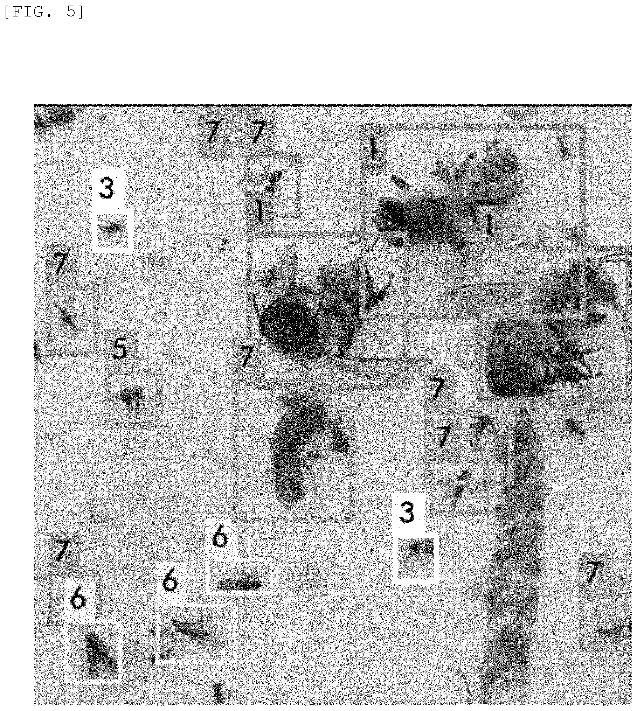

In addition, the processor 15 may identify exposure or proliferation of a natural enemy included in an image sensed by the sensors 21, and transmit a control command for suppressing the natural enemy to the controller 23. To this end, the database 11 may pre-store a critical range of environmental factors in which proliferation of the corresponding natural enemy is suppressed for each type of natural enemy corresponding to the feed insects. Then, the processor 15 may query the database 11 for the identified natural enemy, read out a combination of environmental factors to suppress the growth of the natural enemy, and generate the control command, considering the growth status of the feed insects. When the natural enemy is identified, the processor 15 may notify the manager of the situation regarding the exposure or proliferation of the natural enemy in real time. Thereby, a dangerous situation may be immediately coped with. Automated identification of the feed insects or natural enemies will be described later again with reference to FIG. 5.

The controller 23 controls the growth of feed insects on the breeding farm by adjusting a growth control factor corresponding to each environmental factor according to the combination of the environmental factors read from the processor 15.

In terms of supply and demand control, the processor 15 may calculate the difference between the expected demand input through the input unit 13 and the amount of insects to be supplied from the current breeding farm, calculate remaining days to the input shipment time, and calculate the growth rate of the feed insects required from the current breeding farm based on the difference and the remaining days. Promoting only the growth of feed insects is not the best way. This is because the target of the present disclosure is "feed insects," and the feed insects should be delivered to a feed factory at an appropriate growth point so as to be processed into feed. Accordingly, it is necessary to release grown feed insects in a timely manner in response to a request for supply of the feed insects, which are raw materials. In consideration of this constraint, the embodiments of the present disclosure control the growth rate of feed insects to be increased or decreased according to the expected demand amount and the growth rate or expected supply amount of insects on the current breeding farm.

In consideration of the calculated growth rate, the processor 15 may fix factors that are not involved in the growth of the feed insects among the environmental factors detected through the sensors 21, changing the factors that can change the growth of the feed insects, search the database 11, and read the combination of the environmental factors corresponding to the breeding farm. When there are various environmental factors involved in the growth of insects, a situation in which not all environmental factors can be controlled to ideal values may happen due to changes in seasons, physical constraints on the breeding farm, and the like. In this case, by changing the control values of the remaining factors while fixing the control values of the factors that cannot be significantly involved in the growth of the feed insects (or factors that cannot be controlled), a second best combination of values stored in the database 11 may be derived.

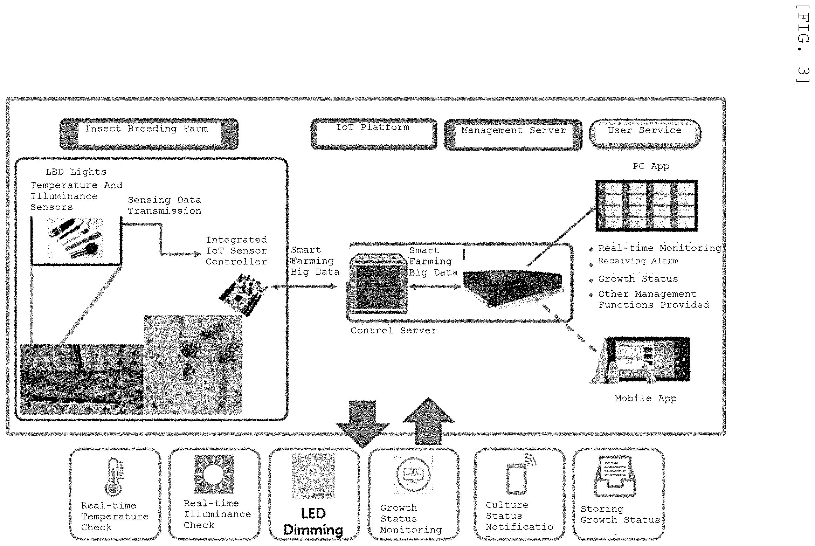

FIG. 3 is a view showing an overall outline of a breeding system including the breeding control device for insects of FIG. 2 according to an embodiment of the present disclosure.

The insect breeding farm is equipped with sensors capable of measuring various environmental factors such as temperature, humidity, and illuminance as well as LED lighting, and the detected measured values are transmitted to a server through an integrated IoT sensor controller. Since a combination of environmental factors related to the growth is pre-stored in the database on the server, an appropriate control command for the breeding farm may be issued based thereon. For example, an LED lighting control technique for a light wavelength band suitable for insect growth may be utilized. When the indoor temperature rises rapidly or the air quality deteriorates in summer, a ventilation fan may be hastily operated to prevent insects from dying.

A series of monitoring and control situations as described above may be provided to a user terminal (PC or mobile device) in real time. In particular, when unusual matter that is harmful to the growth status is found, it is immediately announced such that measures are taken at the early stage. To this end, use of image recognition technology is required. Thus, abnormal behavior of insects or occurrence of natural enemies may be identified/determined from the images of the inside of the breeding farm input through an image sensor such that a corresponding action can be immediately taken.

For a vast amount of data collected from the sensors, big data-based technology is utilized to address performance and scalability issues that occur as the amount of data increases. Therefore, the entire architecture of the breeding control system may be configured based on the Hadoop or NoSQL-based architecture, which is used in the big data environment. In addition, optimized system architecture may be configured considering the use of R, Mahout, Solr, etc. for data analysis.

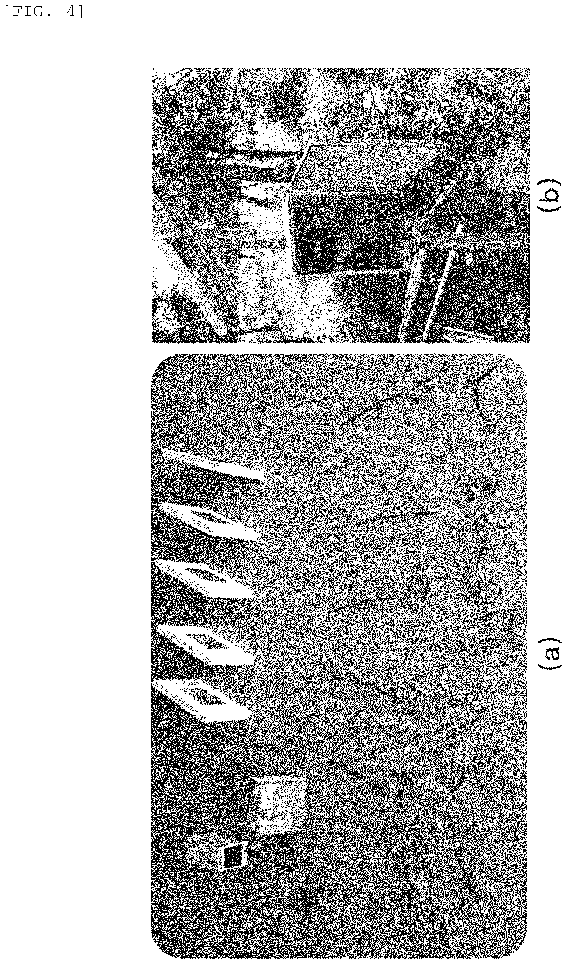

FIG. 4 exemplarily shows sensors and communication means that can be used in a breeding control device for insects according to an embodiment of the present disclosure. As a prototype, a data collection and transmission device is being developed.

As described above, embodiments of the present disclosure provide a system for monitoring of insect diseases and early response by applying IoT technology to the breeding farm, and provide an alert issuing system capable of remotely recognizing the environmental factors inside the insect breeding farm in real time to allow the situation to be coped with promptly. As shown in the figure, prepared sensor devices may be installed at various places in individual breeding spaces on the breeding farm, and may measure environmental factors and transmit the measured factors to the server through the communication means. Thereby, centralized monitoring may be implemented and a corresponding control command may be transmitted to the breeding farm. Accordingly, precise growth control may be performed for each breeding space.

FIG. 5 illustrates the process of identifying and classifying insects adopted according to embodiments of the present disclosure.

As briefly described above, a technique for monitoring abnormal behavior of insects or appearance of natural enemies in the insect breeding farm or automatically analyzing the images is required. For example, it is necessary to take emergency measures such as issuing an alarm when harmful insects such as mites invade, or to monitor hatching and abnormal behaviors such as escape from the breeding farm and notify a manager of the same in real time.

To this end, the processor may specify a movement area from an image sensed through the sensors, and compare the specified movement area with a preset size, color, or movement pattern of feed insects or a natural enemy corresponding to the feed insects, or a combination thereof in order to identify an insect or natural enemy corresponding to the movement area. Then, the kind of the identified insect or natural enemy may be determined, the population of the identified insect or natural enemy may be counted by the kind, the size of the insect or natural enemy may be measured, or the degree of movement of the insect or natural enemy may be quantified. Thereby, detailed information about the growth status may be extracted.

Referring to FIG. 5, the same number may be labeled for individuals classified into the same kind of insects using image recognition technology, and the number of individuals of the same kind may be identified by counting the insects assigned the same number. In terms of implementation, for insect identification and tracking using image recognition and classification technology, elemental technologies such as image recognition using a convolutional neural network (CNN) and moving object tracking using a Kalman filter may be utilized.

When the specified movement area matches only a part of preset characteristic information about the feed insect, the processor checks whether the area matches additional information indicating the diseased state of the feed insect to determine whether the feed insect has a disease. For example, when it is confirmed that the insect is a growing feed insect based on the size and color of the insect, but the movement pattern is different from previously stored characteristic information, additional information indicating a disease may be checked and the insect may be determined to be in a diseased state.

When the counted number of individuals for each kind of insect is greater than or equal to a space threshold in an allocated space in the breeding farm, the processor may calculate an additional space required for the growth of the insects and notify the manager of the calculated space. In this way, the risk of inhibiting the growth of insects or causing the insects to die due to lack of breeding space may be prevented.

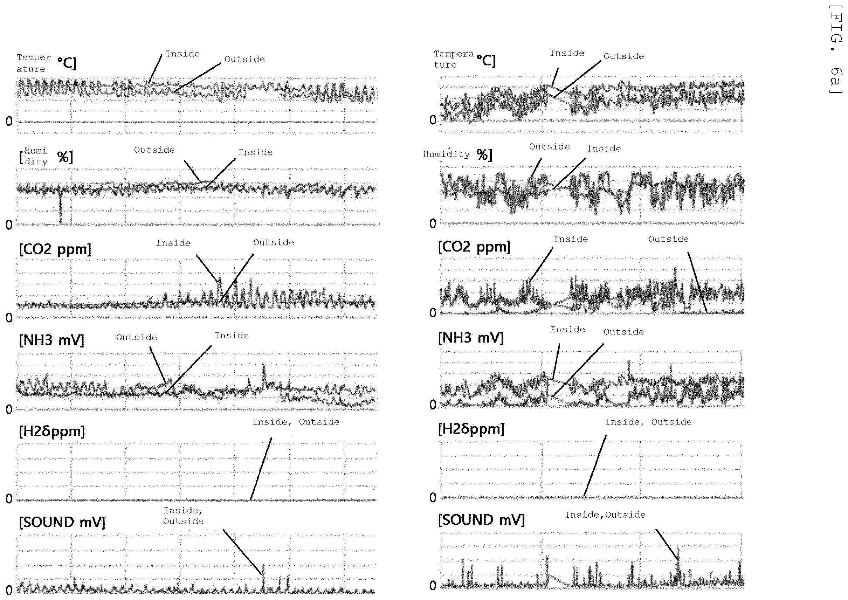

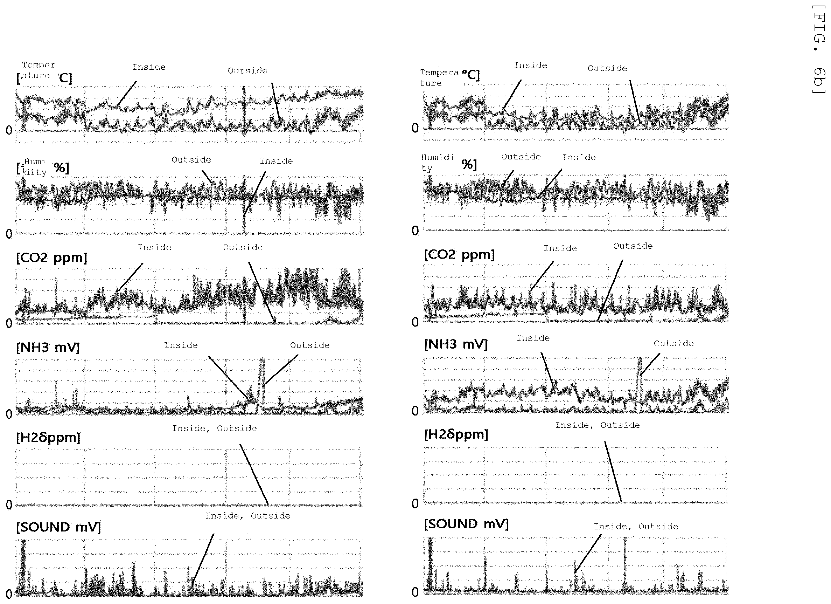

FIGS. 6A and 6B depict the results of monitoring of environmental factors when damage is caused by natural enemies. The blue curves represent the values measured inside the breeding farm, and the red curves represent the values measured outside the breeding farm. Each curve represents values by measuring various growth environmental factors such as temperature and humidity, and the temperature/humidity environment for survival and growth of feed insects was adjusted over time.

In particular, FIG. 6B shows a typical pattern that appears when there is damage caused by mites and ticks. It can be seen from the figure that the temperature control failure on the breeding farm is prominent. In other words, the figure shows that in order to promote the growth of insects, not only control of environmental factors for the insects, but also environmental factors through which natural enemies are activated should be considered. If the optimal combination of environmental factors that promote the growth of feed insects activates natural enemies as well, a more conservative approach is needed in terms of growth management. Even a second best combination of environmental factors for insect growth may be selected as long as it can suppress the occurrence of natural enemies.

To this end, in embodiments of the present disclosure, a critical range of environmental factors in which the growth of the corresponding natural enemy is suppressed for each kind of natural enemy corresponding to the feed insect may be pre-stored in the database, and the database may be queried for the identified natural enemy through the processor. Then, a combination of environmental factors that suppresses the growth of the natural enemy is read. Then, a control command is generated in consideration of the growth status of the feed insects. That is, the breeding farm is controlled through a combination of environmental factors that considers both the growth of feed insects and suppression of natural enemies simultaneously.

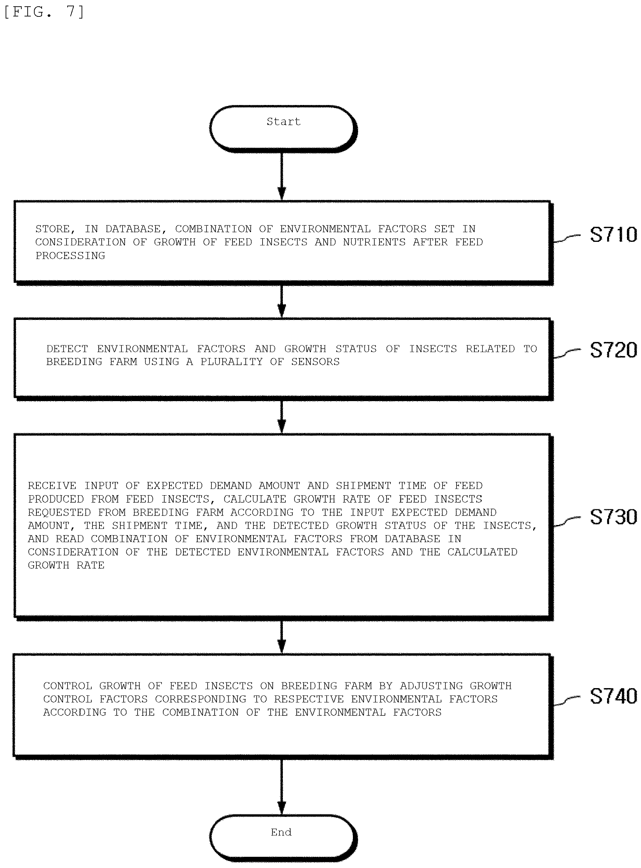

FIG. 7 is a flowchart illustrating a method for controlling the breeding of insects according to an embodiment of the present disclosure, in which the functions/operations of the respective components of the breeding control device described above with reference to FIG. 2 are reconfigured in time series. Therefore, in order to avoid redundant description, only an outline thereof is briefly described herein.

In operation S710, the breeding control device stores, in the database, a combination of environmental factors that is set in consideration of the growth of feed insects and nutrients after feed processing.

In operation S720, the breeding control device detects environmental factors and the growth status of the insects related to a breeding farm where the feed insects are bred, using a plurality of sensors.

In operation S730, the breeding control device receives an input of the expected demand amount and shipment time of the feed produced from the feed insects, calculates the growth rate of the feed insects requested from the current breeding farm according to the input expected demand amount, the shipment time, and the detected growth status of the insects, and reads the combination of the environmental factors from the database in consideration of the environmental factors detected through the sensors and the calculated growth rate.

More specifically, in operation S730 of reading the combination of environmental factors, the difference between the input expected demand and the amount of insects to be supplied from the current breeding farm may be calculated, and the remaining days to the input shipment time may be calculated. Then, the growth rate of feed insects required from the current breeding farm may be calculated based on the difference and the remaining days. In addition, in operation S730 of reading the combination of environmental factors, in consideration of the calculated growth rate, factors that are not involved in the growth of the feed insects among the environmental factors detected through the sensors may be fixed, and the database may be searched while changing the factors that can change the growth of the feed insects. Then, the combination of the environmental factors for the breeding farm may be read.

In operation S730 of reading the combination of environmental factors, a movement area from an image sensed through the sensors may be specified, and the specified movement area may be compared with a preset size, color, or movement pattern of feed insects or a natural enemy corresponding to the feed insects, or a combination thereof in order to identify an insect or natural enemy corresponding to the movement area. Then, the kind of the identified insect or natural enemy may be determined, the population of the identified insect or natural enemy may be counted by the kind, the size of the insect or natural enemy may be measured, or the degree of movement of the insect or natural enemy may be quantified. Thereby, detailed information about the growth status may be extracted. In the operation of identifying the insect or natural enemy, when the specified movement area matches only a part of preset characteristic information about the feed insect, it may be checked whether the area matches additional information indicating the diseased state of the feed insect to determine whether the feed insect has a disease. Furthermore, when the counted number of individuals for each kind of insect is greater than or equal to a space threshold in an allocated space on the breeding farm, an additional space required for the growth of the insects may be calculated and the manager may be notified thereof.

In operation S740, the breeding control device controls the growth of feed insects on the breeding farm by adjusting the growth control factors corresponding to the respective environmental factors according to the combination of the environmental factors read in operation S730.

The breeding control method of FIG. 7 may further include identifying exposure or proliferation of a natural enemy included in the image sensed by the sensors and transmitting a control command for suppressing the natural enemy to the breeding farm. The method may further include storing, in the database, a critical range of an environmental factor for suppressing proliferation of the natural enemy for each kind of natural enemy corresponding to the feed insects. Thus, in the operation of transmitting the control command to the breeding farm, the database may be queried for the identified natural enemy, and a combination of environmental factors that suppresses the growth of the natural enemy may be read. Then, a control command is generated in consideration of the growth status of the feed insects.

According to embodiments of the present disclosure, the competitiveness of the insect industry may be enhanced by controlling the growth rate of insects in consideration of the supply and demand of feed insects, as well as environmental factors for promotion of insect breeding. Also, high-quality feed insects may be supplied by controlling the environmental factors necessary for growth from the perspective of food and nutrition after feed processing. In addition, by monitoring the activity of insects or natural enemies through various sensors and image identification technology provided in the breeding farm and performing automated real-time control, early prediction of insect growth status and a proper action may be enabled.

The present disclosure may be implemented as computer-readable code on a computer-readable recording medium. The computer-readable recording medium includes all kinds of recording devices in which data that may be read by a computer system is stored.

Examples of computer-readable recording media include ROM, RAM, CD-ROM, magnetic tapes, floppy disks, and optical data storage devices. The computer-readable recording medium may be distributed to a computer system connected over a network, and computer-readable code may be stored and executed thereon in a distributed manner. Functional programs, code, and code segments for implementing the method described above may be easily inferred by programmers in the art to which the embodiments pertain.

Various embodiments of the present disclosure have been mainly discussed. Those of ordinary skill in the art to which the present disclosure pertains will understand that modifications can be made to the present disclosure without departing from the essential features of the present disclosure. Therefore, the disclosed embodiments are to be considered as illustrative rather than restrictive. The scope of the present disclosure is to be defined by the claims rather than the foregoing description, and all differences within the scope equivalent thereto should be construed as being included in the present disclosure.

### Reference Numerals

- 10:: S/W module of breeding control device
- 11:: Database
- 13:: Input unit
- 15:: Processor
- 20:: H/W module of breeding control device
- 21:: Sensor
- 23:: Controller

## Claims

1. A breeding control device comprising:
a database configured to store a combination of environmental factors set in consideration of growth of feed insects and nutrients after feed processing;
a plurality of sensors configured to detect environmental factors and a growth status of the insects related to a breeding farm for breeding the feed insects;
a processor configured to:
receive an input of an expected demand amount and shipment time of feed produced from the feed insects;
calculate a growth rate of the feed insects requested from the breeding farm according to the input expected demand amount, the shipment time, and the detected growth status of the insects; and
read the combination of the environmental factors from the database in consideration of the environmental factors detected through the sensors and the calculated growth rate; and
a controller configured to control the growth of the feed insects on the breeding farm by adjusting a growth control factor corresponding to each of the environmental factors according to the combination of the environmental factors read from the processor.

2. The breeding control device of claim 1, wherein the sensors comprise:
an environmental sensor configured to detect at least one environmental factor among temperature, illuminance, humidity, and air quality involved in the growth of the insects on the breeding farm; and
a growth sensor configured to detect a growth status of at least one of a kind, size, color, movement, and number of the insects.

3. The breeding control device of claim 1, wherein the processor identifies exposure or proliferation of a natural enemy included in an image sensed by the sensors, and transmits a control command for suppressing the natural enemy to the controller.

4. The breeding control device of claim 3, wherein the database pre-stores a critical range of the environmental factors for suppressing growth of a corresponding natural enemy according to each kind of natural enemy corresponding to the feed insects, and
wherein the processor queries the database for the identified natural enemy, reads a combination of environmental factors for suppressing the growth of the natural enemy, and generates the control command in consideration of the growth status of the feed insects.

5. The breeding control device of claim 3, wherein, when the natural enemy is identified, the processor notifies a manager of a situation related to the exposure or proliferation of the natural enemy in real time.

6. The breeding control device of claim 1, wherein the processor specifies a movement area from the image sensed through the sensors,
wherein the processor is configured to:
identify insects or natural enemies corresponding to the movement area by comparing the specified movement area with a preset size, color, or movement pattern of the feed insects or the natural enemy corresponding to the feed insects, or a combination thereof; and
extract detailed information about the growth status by:
determining a kind of the identified insect or natural enemy;
counting the number of the identified insects or natural enemies for each determined kind of insects or natural enemies;
measuring a size of the insects or natural enemies; or
quantifying a degree of movement of the insects or natural enemies.

7. The breeding control device of claim 6, wherein, when the specified movement area matches only a part of preset characteristic information about the feed insects, the processor checks whether the area matches additional information indicating a diseased state of the feed insects and determines whether the feed insects have a disease.

8. The breeding control device of claim 6, wherein, when the counted number of the insects for each kind of the insects is greater than or equal to a space threshold in an allocated space on the breeding farm, the processor calculates an additional space required for growth of the insects and notifies a manager of the calculated space.

9. The breeding control device of claim 1, wherein the processor is configured to:
calculate a difference between the input expected demand and an amount of insects to be supplied from the breeding farm;
calculate remaining days to the input shipment time; and
calculate a growth rate of the feed insects required from the breeding farm based on the difference and the remaining days.

10. The breeding control device of claim 1, wherein, in consideration of the calculated growth rate, the processor is configured to:
fix factors not involved in the growth of the feed insects among the environmental factors detected through the sensors;
search the database while changing the factors capable of changing the growth of the feed insects; and
read the combination of the environmental factors for the breeding farm.

11. The breeding control device of claim 1, wherein the database stores a combination of environmental factors experimentally set for growth of the feed insects such that target remaining nutrients are maximized after the feed processing through roasting and drying.

12. A breeding control method comprising:
storing, in a database, a combination of environmental factors set in consideration of a growth of feed insects and nutrients after feed processing;
detecting environmental factors and a growth status of the insects related to a breeding farm for breeding the feed insects using a plurality of sensors;
receiving an input of an expected demand amount and shipment time of feed produced from the feed insects, calculating a growth rate of the feed insects requested from the breeding farm according to the input expected demand amount, the shipment time, and the detected growth status of the insects, and reading the combination of the environmental factors from the database in consideration of the environmental factors detected through the sensors and the calculated growth rate; and
controlling the growth of the feed insects on the breeding farm by adjusting a growth control factor corresponding to each of the environmental factors according to the read combination of the environmental factors.

13. The method of claim 12, further comprising:
identifying exposure or proliferation of a natural enemy included in an image sensed by the sensors; and
transmitting a control command for suppressing the natural enemy to the breeding farm.

14. The method of claim 13, further comprising:
pre-storing, in the database, a critical range of the environmental factors for suppressing a growth of a corresponding natural enemy according to each kind of natural enemy corresponding to the feed insects,
wherein the transmitting of the control command to the breeding farm comprises:
querying the database for the identified natural enemy;
reading a combination of environmental factors for suppressing the growth of the natural enemy; and
generating the control command in consideration of the growth status of the feed insects.

15. The method of claim 12, wherein the reading of the combination of the environmental factors comprises:
specifying a movement area from the image sensed through the sensors;
identifying insects or natural enemies corresponding to the movement area by comparing the specified movement area with a preset size, color, or movement pattern of the feed insects or the natural enemy corresponding to the feed insects, or a combination thereof; and
extracting detailed information about the growth status by:
determining a kind of the identified insect or natural enemy;
counting the number of the identified insects or natural enemies for each determined kind of insects or natural enemies;
measuring a size of the insects or natural enemies; or
quantifying a degree of movement of the insects or natural enemies.

16. The method of claim 15, wherein, when the specified movement area matches only a part of preset characteristic information about the feed insects, the identifying of the insects or natural enemies comprises:
checking whether the area matches additional information indicating a diseased state of the feed insects and determining whether the feed insects have a disease.

17. The method of claim 15, further comprising:
when the counted number of the insects for each kind of the insects is greater than or equal to a space threshold in an allocated space on the breeding farm, calculating an additional space required for growth of the insects and notifying a manager of the calculated space.

18. The method of claim 12, wherein the reading of the combination of the environmental factors comprises:
calculating a difference between the input expected demand and an amount of insects to be supplied from the breeding farm;
calculating remaining days to the input shipment time; and
calculating a growth rate of the feed insects required from the breeding farm based on the difference and the remaining days.

19. The method of claim 12, wherein, in consideration of the calculated growth rate, the reading of the combination of the environmental factors comprises:
fixing factors not involved in the growth of the feed insects among the environmental factors detected through the sensors;
searching the database while changing the factors capable of changing the growth of the feed insects; and
reading the combination of the environmental factors for the breeding farm.

20. A computer-readable recording medium having a program for executing the method of claim 12 in a computer recorded thereon.
